# EUROPEAN PATENT APPLICATION

(11) **EP 2 749 288 A2**
(43) Date of publication of application: **02.07.2014**
(21) Application number: 13199628.2
(22) Date of filing: 27.12.2013
(51) Int. Cl.: A61K 36/45

(54) **Capsule and composition comprising seed oil**

(30) Priority: 28.12.2012 FI 20126389
(71) Applicant: Aromtech Ltd, 95401 Tornio (FI)
(72) Inventor: Judin, Vesa-Pekka, 28540 Pori (FI); Larmo, Petra, 95400 Tornio (FI)
(74) Representative: Turun Patenttitoimisto Oy

(57) **Abstract**

The invention relates to a capsule consisting of a closed soluble soft shell and within the closed soft shell a composition consisting of seed oil. The seed oil is selected from lingonberry seed oil, blueberry seed oil, bilberry seed oil, or any of their mixture and which composition comprises γ-tocotrienol at least 0.2 mg/g of seed oil and α-linolenic acid at least 300 mg/g of seed oil. The invention relates also to a composition for use in skin lightening and/or for increasing skin density by oral administration.

## Description

The invention relates to a capsule and a composition comprising seed oil according to the preambles of the enclosed independent claims.

Hyperpigmentation, chloasmata and senile spots are common, but unwanted signs of ageing of the skin. Prevailing culture prefers even skin tones, which are considered appealing and pleasing. In Asian, African and Hispanic cultures there is also a strong desire for lighter skin tones. Furthermore, it is known that ultraviolet (UV) exposure often results in hyperpigmentation, dyspigmentation, and uneven skin tones, and in general darkens the skin tone. Therefore a great demand exists for skin lightening formulations and for formulations smoothing the skin tone.

Hydroquinone and kojic acid may be used topically as skin lightening ingredients because they function as inhibitors of melanin synthesis. In addition to safety concerns, such as cancer risk and skin irritation, these ingredients are extremely unstable and lose their activity in the end product soon after opening of the package. To overcome the problem, some derivatives of these compounds such as kojic dipalmitate have been used in topical formulations. However the efficacy of these derivatives has not been fully demonstrated. All trans-retinoic acid, alpha hydroxy acid and hydroxy acids from synthetic and natural sources are not effective in reducing melanin synthesis but are often used in combination with other lightening ingredients to improve the overall appearance of skin.

Document WO 2006/117430 discloses a composition comprising at least one triglyceride of at least one unsaturated fatty acid which is used for manufacture of a topical product for skin lightening. The skin care composition comprises a dermatologically acceptable additive and pure seed oil derived from a plant selected from the group Rubus, Vaccinium, Oxycoccus, Fragaria, Hipphophae, Echium, Hordeum, Avena, or a mixture thereof.

However, skin lightening by using topical compositions may have certain drawbacks. It is difficult to administer the skin lightening ingredient accurately, so that a sufficient effective dose is applied on the skin and simultaneously overdosing is avoided. Also, some consumer groups, for example males, may be less willing to use topical formulations. Consequently, there exists a desire for alternative ways for skin lightening.

Also skin density decreases with age and/or as a result of prolonged exposure for ultraviolet radiation. Decreased skin density results in wrinkles, which are considered as cosmetic defects by many, especially in western cultures which prefer youthful appearance. Thus a large number of people are suffering problems associated with uneven skin colour and/or decreased skin density. Preferably, same products could also be used both for skin lightening and for increasing the skin density.

### OBJECTS AND SUMMARY OF THE INVENTION

An object of the present invention is to minimise or even completely eliminate the problems existing in the prior art.

One object of the present invention is thus to provide a skin lightening product that is effective and safe for the user.

Another object is to provide a skin lightening product which is easy to use and has good storage stability.

Typical capsule according to the present invention consists of
- a closed soluble soft shell and within the closed soft shell
- a composition consisting of seed oil which is selected from lingonberry seed oil, blueberry seed oil, bilberry seed oil or any of their mixture and which composition comprises γ-tocotrienol at least 0.2 mg/g of seed oil and α-linolenic acid at least 300 mg/g of seed oil.

Typical composition comprising a seed oil which is selected from lingonberry seed oil, blueberry seed oil, cranberry seed oil or any of their mixture, the composition comprising γ-tocotrienol and α-linolenic acid is for use in skin lightening and/or for increasing skin density by orally administering the composition in such amount that a daily dosage of γ-tocotrienol is at least 0.4 mg and of α-linolenic acid is at least 600 mg.

Now it has been surprisingly found out that seed oils of lingonberry, blueberry, bilberry and cranberry are effective for skin lightening and/or for increasing skin density even when they are administered orally. Furthermore, the seed oils have beneficial effects in reducing skin wrinkles, improving skin elasticity and skin moisture. A composition comprising at least one of the said seed oils and comprising effective amounts of γ-tocotrienol, α-linolenic acid and optionally linoleic acid may be easily administered in form of soft shell capsules, which are soluble in human digestive system. This mode of administration ensures the attaining of the sufficient daily dosage with greater accuracy. Furthermore, oral administration is more acceptable for all possible consumer groups. Capsule also protects the seed oil and the effective ingredients from air exposure and oxidations, which improves the storage life of the product. Surprisingly it was also found out that no other ingredients than seed oil are necessary for obtaining the positive results in increasing skin density and skin lightening.

According to one embodiment of the invention the seed oil comprises linoleic acid at least 260 mg/g of seed oil.

The seed oils, which are useful in manufacturing oral capsules for skin lightening and/or for increasing the skin density would typically contain a total content of linoleic acid [18:2(n - 6)] and α-linolenic acid [18:3(n - 3)] at levels of 20 - 90 weight-%, preferably 60 - 90 weight-%, of the total fatty acids. One preferred fatty acid composition of the oils contains linoleic and α-linolenic acids at ratios from 2:1 to 1:2. According to one embodiment, said ratio is around 1:1. Other ratios may naturally also be used.

General structure of linoleic acid is shown below:

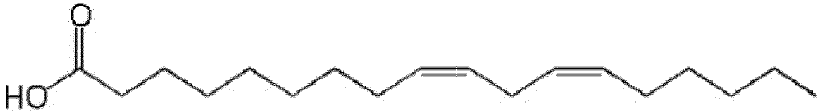

General structure of α-linolenic acid is shown below:

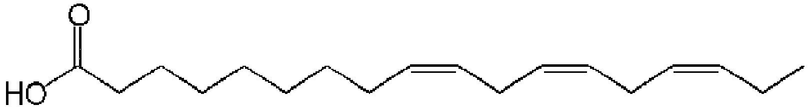

In the present context, linoleic acid does not comprise conjugated linoleic acids, which are structurally different fatty acids and also from different sources, such as from dairy products and synthetic sources.

The seed oils, which are useful in manufacturing oral capsules for skin lightening and/or for increasing the skin density typically contain γ-tocotrienol. The concentration of γ-tocotrienol is typically in the range of 0.02 - 0.18 weight-%, preferably 0.08 - 0.18 weight-%. General structure of γ-tocotrienol is shown below:

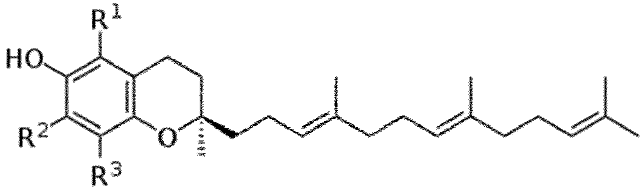

A beneficial composition of γ-tocotrienol, α-linolenic acid and optionally linoleic acid may be found, for example, in the seed oils of the lingonberry, blueberry, bilberry or cranberry, the seed oil of lingonberry being preferred. Also mixtures of these seed oils may be used. Mixture can comprise seed oils from two or more different plant genera, or seed oils from two or more different plant species of the same genus. Blueberry, bilberry, cranberry and lingonberry seed oils are useful in the present invention because of the combination of the high content of γ-tocotrienol, α-linolenic and optionally linoleic acids.

According to one embodiment of the invention the seed oil comprises
- γ-tocotrienol 0.2 - 1.8 mg/g of seed oil, preferably 0.4 - 1.8 mg/g of seed oil, more preferably 0.8 - 1.8 mg/g of seed oil, and/or
- α-linolenic acid 300 - 570 mg/g of seed oil, preferably 340 - 570 mg/g of seed oil, more preferably 370 - 570 mg/g of seed oil, and/or
- linoleic acid 260 - 430 mg/g of seed oil, preferably 260 - 425 mg/g of seed oil, more preferably 260 - 420 mg/g of seed oil.

The capsule may comprise the composition comprising seed oil in amount of 0.01 - 99.99 weight-%, typically 40 - 99 weight-%, more typically 60 - 99 weight-%, based on the total weight of the capsule.

According to one embodiment the composition in the capsule may comprise an added antioxidant agent, such as antioxidant rosemary extract, natural α-tocopherol and/or carotenoids in an oil vehicle, as an additive. Rosemary extract is derived from *Rosmarinus officinalis* L. Carotenoids are preferably oxygen free carotenoids, such as β-carotene or lycopene. The total amount of the antioxidant agent, natural α-tocopherol and/or carotenoids is typically less than 1 weight-%, based on the weight of the seed oil.

According to one embodiment of the invention the blueberry seed oil or bilberry seed oil is derived from seeds of *Vaccinium angustifolium, Vaccinium boreale, Vaccinium bracteatum, Vaccinium cespitosum, Vaccinium caesariense, Vaccinium corymbosum, Vaccinium darrowii, Vaccinium elliottii, Vaccinium formosum, Vaccinium fuscatum, Vaccinium hirsutum, Vaccinium koreanum, Vaccinium myrsinites, Vaccinium myrtilloides, Vaccinium pallidum, Vaccinium simulatum, Vaccinium tenellum, Vaccinium virgatum, Vaccinium myrtillus, Vaccinium uliginosum L., Vaccinium caespitosum, Vaccinium deliciosum, Vaccinium membranaceum,* and/or *Vaccinium ovalifolium.* According to one preferred embodiment of the invention the blueberry seed oil is obtained from seeds of *Vaccinium myrtillus, Vaccinium corymbosum, Vaccinium angustifolium,* and/or *Vaccinium fuscatum.*

According to one embodiment cranberry seed oil is obtained from seeds of *Vaccinium macrocarpon, Vaccinium oxycoccos, Vaccinium erythrocarpum* and/or *Vaccinium microcarpum.* Among the seed oils of different Vaccinium species, cranberry seed oils are particularly rich in γ-tocotrienol. Therefore they are suitable for use in the present invention.

According to one preferred embodiment of the invention the lingonberry seed oil is obtained from seeds of *Vaccinium vitis-idaea.* Among the seed oils of different Vaccinium species, lingonberry seed oil has a high content of essential α-linolenic acid. Preferably the capsule according to the present invention comprises lingonberry seed oil.

According to one embodiment of the invention the capsule comprises seed oil mixture from blueberry, lingonberry and cranberry. This seed oil mixture may comprise, for example, γ-tocotrienol 0.2 - 1.8 mg/g of seed oil mixture, and α-linolenic acid 300 - 570 mg/g of seed oil mixture, linoleic acid 260 - 430 mg/g of seed oil mixture.

According to another embodiment of the invention the capsule comprises seed oil mixture from lingonberry and cranberry. This seed oil mixture may comprise, for example, γ-tocotrienol 0.4 - 1.8 mg/g of seed oil mixture, preferably 0.8 - 1.8 mg/g of seed oil mixture, α-linolenic acid 340 - 570 mg/g of seed oil mixture, and linoleic acid 260 - 430 mg/g of seed oil.

Seed oils may be obtained by any suitable method. They include, for example, raw, semi-refined and refined oils, isolated by cold pressing and extraction with organic solvents or with CO₂. Also seed oils isolated by mechanical methods may be used. Preferably, seed oil may be obtained by supercritical CO₂ extraction and it is free of any solid matter and organic solvents. Among the different oil manufacturing technologies, supercritical CO₂ extraction provides a good quality due to the high extraction efficiency and the absence of harmful solvent residues in the oils extracted. Thus supercritical CO₂-extracted oils are preferred for use in capsule which is taken orally.

The soft shell of the capsule may be made of plant based raw materials comprising native and modified plant polysaccharides, such as starch, and polyols, such as sorbitol or glycerin. Preferably the soft shell is made solely of plant based raw materials. Typically one capsule comprises at least 0.3 ml seed oil, preferably 0.3 - 1.5 ml, more preferably 0.5 - 1.0 ml, still more preferably 0.5 - 0.7 ml seed oil.

According to one embodiment of the invention the daily dosage of γ-tocotrienol is 0.4 - 3.6 mg, preferably 0.8 - 3.6 mg, more preferably 1.6 - 3.6 mg; of α-linolenic acid is 600 mg - 1140 mg, preferably 680 - 1140 mg, more preferably 740 - 1140 mg; and of linoleic acid is 520 mg - 860 mg.

The present invention is useful in whitening or lightening the overall skin tone, or whitening or lightening age spots or freckles. As the capsules of the present invention are safe to use, they can be used to lighten or whiten the skin of children or adolescents.

### EXPERIMENTAL

The effect of CO₂-extracted lingonberry seed oil on skin was investigated in a clinical study carried out at ISPE- Institute of Skin and Product Evaluation (Milan, Italy) during 2012.

A total of 30 healthy female volunteers were included to the open one-group study. The participants were 35-65 years of age and had hyper-chromatic skin spots on the face. During the study period of 12 weeks they daily consumed 4 capsules (2 g) of lingonberry seed oil. The following parameters were instrumentally measured at the beginning, at 6 weeks and at 12 weeks when the study period ended:
- Skin hydration (corneometric values), measured by using Corneometer CM 825 (Courage & Khazaka), which comprises a square-shaped sensor, area 49 mm², with a special glass covering the front, on a mobile axis, connected to the base unit by a spiral cable. The measurement principle is based on capacitance;
- Skin elasticity, measured by using Cutometer MPA 580 (Courage & Khazaka). Following parameters were considered: R0 describing the maximal deformation of the skin, R6 describing viscoelastic ratio andR2 describing the overall elasticity;
- Skin echogenicity (skin density), measured by using a high-resolution scanner emitting high frequency ultrasound (20 MHz) (Dermascan C® Ver. 3 (Cortex Technology, Danmark);
- Skin roughness, measured by using skin replicas and image analysis, Monaderm. Following parameters were considered: Ra describing average roughness of the profile and Rz describing average maximum roughness;
- Skin colour of hyper-chromatic skin spots, ITA° and L* values. Skin colour was measured using a portable dual channel, reflecting colorimeter with incorporated microcomputer, liquid crystals display and Xenon light source in the measuring head (Chromameter CR-400, Minolta).

At the end of the treatment the volunteers were requested to answer to a self-assessment questionnaire in order to express their opinion about the effect of lingberry seed oil on skin.

The change from baseline at two and three months was analysed with suitable statistical methods, Repeated Measures ANOVA and Bonferroni Test for parametric data. A significance level of 0.05 was used throughout.

The instrumental evaluations, performed at the beginning, after 6 weeks and at the end of the 12 weeks of treatment showed a significant improvement for the following parameters:
- a statistically significant increase in skin hydration values, both on the face and on the forearm, both after 6 and 12 weeks of treatment;
- a statistically significant improvement of the skin elasticity showed by:
   - a statistically significant decrease in maximal deformation values R0 both after 6 weeks and at the end of the treatment;
   - a statistically significant increase in overall elasticity values R2 after 12 weeks of treatment;
- a statistically significant decrease in average roughness Ra both after 6 and 12 weeks of treatment, and average maximum roughness Rz at the end of the treatment;
- a statistically significant increase in skin echogenicity (skin density) values both after 6 and 12 weeks of treatment.

In addition there was a trend indicating whitening of hypercromatic skin spots from baseline to the end of intervention.

The results concerning effect of oral lingonberry seed oil on skin density are presented in Table 1. The results concerning the colour of hyperchromatic spots are presented in Tables 2 and 3.

**Table 1. Echogenicity, expressed as percentage, of skin at baseline, at 6 weeks and at 12 weeks.**

| T₀ | T_{6weeks} | T_{12weeks} | Variation T_{6weeks} - T₀ | Variation T_{12weeks} - T₀ |
|---|---|---|---|---|
| mean 36.34 | mean 37.76 | mean 39.89 | 1.42 | 3.55 |
| std.dev.: 5.11 | std.dev.: 5.15 | std.dev.: 5.92 | p<0.05 | p<0.0001 |

The results of Table 1 indicate significant, P<0.05, increase in the density of the skin during the lingonberry seed oil intervention

**Table 2. L* parameter of skin hyperchromatic spots at baseline, at 6 weeks and at 12 weeks.**

| T₀ | T_{6weeks} | T_{12weeks} | Variation (%) T_{6weeks} - T₀ | Variation (%) T_{12weeks} - T₀ |
|---|---|---|---|---|
| mean 59.39 | mean 59.41 | mean 59.53 | 0.02 (0.03%) | 0.14 (0.2%) |
| std.dev.: 1.95 | std.dev.: 1.94 | std.dev.: 1.82 | p>0.05 | p>0.05 |

The results of Table 2 indicate a trend (P>0.05) of skin whitening, i.e. increase of L* value, during the lingoberry seed oil intervention.

**Table 3. Individual Tipology Angle, ITA° parameter of skin hyperchromatic spots at baseline, at 6 weeks and at 12 weeks.**

| T₀ | T_{6weeks} | T_{12weeks} | Variation T_{6weeks} - T₀ | Variation T_{12weeks} - T₀ |
|---|---|---|---|---|
| mean 26.7 | mean 27.1 | mean 27.4 | 0.4 | 0.7 |
| std.dev.: 5.3 | std.dev.: 5.0 | std.dev.: 4.9 | p>0.05 | p>0.05 |

The results of Table 3 indicate a trend (P>0.05) of skin whitening, i.e. increase of ITA° value, during the lingonberry seed oil intervention

In conclusion, the study suggests significant beneficial effects of intake of lingonberry seed oil on skin, and a beneficial trend for the whitening of hyperchromatic spots of skin.

Even if the invention was described with reference to what at present seems to be the most practical and preferred embodiments, it is appreciated that the invention shall not be limited to the embodiments described above, but the invention is intended to cover also different modifications and equivalent technical solutions within the scope of the enclosed claims.

## Claims

1. A capsule consisting of
- a closed soluble soft shell and within the closed soft shell
- a composition consisting of seed oil which is selected from lingonberry seed oil, blueberry seed oil, bilberry seed oil or any of their mixture and which composition comprises γ-tocotrienol at least 0.2 mg/g of seed oil and α-linolenic acid at least 300 mg/g of seed oil.

2. Capsule according to claim 1, **characterised in that** the seed oil comprises linoleic acid at least 260 mg/g of seed oil.

3. Capsule according to claim 2, **characterised in that** the seed oil comprises
- γ-tocotrienol 0.2 - 1.8 mg/g of seed oil, preferably 0.4 - 1.8 mg/g of seed oil, more preferably 0.8 - 1.8 mg/g of seed oil, and/or
- α-linolenic acid 300 - 570 mg/g of seed oil, preferably 340 - 570 mg/g of seed oil, more preferably 370 - 570 mg/g of seed oil, and/or
- linoleic acid 260 - 430 mg/g of seed oil, preferably 260 - 425 mg/g of seed oil, more preferably 260 - 420 mg/g of seed oil.

4. Capsule according to claim 1, **characterised in that** capsule comprises the composition consisting of seed oil in amount of 0.01 - 99.99 weight-%, typically 40 - 99 weight-%, more typically 60 - 99 weight-%, based on the total weight of the capsule.

5. Capsule according to claim 1, **characterised in that** the blueberry seed oil or bilberry seed oil is obtained from seeds of *Vaccinium myrtillus, Vaccinium corymbosum, Vaccinium angustifolium,* and/or *Vaccinium fuscatum.*

6. Capsule according to claim 1, **characterised in that** the lingonberry seed oil is obtained from seeds of *Vaccinium vitis-idaea.*

7. Capsule according to claim 1, **characterised in that** the composition consist of a seed oil mixture from blueberry, lingonberry and cranberry, the seed oil mixture comprising γ-tocotrienol 0.2 - 1.8 mg/g of seed oil mixture, α-linolenic acid 300 - 570 mg/g of seed oil mixture, and linoleic acid 260 - 430 mg/g of seed oil mixture.

8. Capsule according to any of preceding claims 1 - 7, **characterised in that** the seed oil is obtained by supercritical CO₂ extraction and is free of any solid matter and organic solvents.

9. Capsule according to claim 1, **characterised in that** the soft shell is made of plant based raw materials comprising native and modified plant polysaccharides, such as starch, and polyols, such as sorbitol or glycerin.

10. Capsule according to claim 1, **characterised in that** it comprises at least 0.3 ml seed oil, preferably 0.3 - 1.5 ml, more preferably 0.5 - 1.0 ml, still more preferably 0.5 - 0.7 ml seed oil.

11. A composition comprising a seed oil which is selected from lingonberry seed oil, blueberry seed oil, cranberry seed oil or any of their mixture, the composition comprising γ-tocotrienol and α-linolenic acid, for use in skin lightening and/or for increasing skin density by orally administering the composition in such amount that a daily dosage of γ-tocotrienol is at least 0.4 mg and of α-linolenic acid is at least 600 mg.

12. Composition according to claim 11, **characterised in that** the composition comprises linoleic acid, whereby the daily dosage of linoleic acid is at least 520 mg.

13. Composition according to claim 11, **characterised in that** the daily dosage
- of γ-tocotrienol is 0.4 - 3.6 mg, preferably 0.8 - 3.6 mg, more preferably 1.6 - 3.6 mg,
- of α-linolenic acid is 600 mg - 1140 mg, preferably 680 - 1140 mg, more preferably 740 - 1140 mg, and
- of linoleic acid is 520 mg - 860 mg, preferably 520 - 850 mg, more preferably 520 - 840 mg.
